(19) Europäisches Patentamt
European Patent Office
Office européen des brevets

(11) Veröffentlichungsnummer **0 029 139**
**A1**

(12) # EUROPÄISCHE PATENTANMELDUNG

(21) Anmeldenummer: **80106576.4**

(22) Anmeldetag: **25.10.80**

(51) Int. Cl.³: **C 07 D 233/58**, A 61 K 31/415

(30) Priorität: **06.11.79 DE 2944663**

(43) Veröffentlichungstag der Anmeldung: **27.05.81 Patentblatt 81/21**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Lieb, Folker, Dr., Alfred-Kubin-Strasse 1, D-5090 Leverkusen 1 (DE)**
Erfinder: **Oediger, Hermann, Dr., Roggendorfstrasse 51, D-5000 Koeln 80 (DE)**
Erfinder: **Busse, Wolf-Dieter, Dr., Pahlkestrasse 65, D-5600 Wuppertal 1 (DE)**
Erfinder: **Seuter, Friedel, Dr., Moospfad 16, D-5600 Wuppertal 1 (DE)**
Erfinder: **Perzborn, Elisabeth, Dr., Sadowastrasse 65, D-5600 Wuppertal 1 (DE)**

(54) **Imidazolderivate, Verfahren zu ihrer Herstellung sowie ihre Verwendung in Arzneimitteln.**

(57) Imidazolderivate der allgemeinen Formel

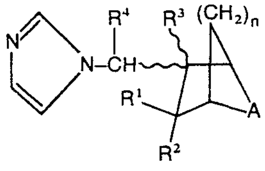

in welcher

$R^1$, $R^2$, $R^3$ und $R^4$ für Wasserstoff oder einen gegebenenfalls substituierten Alkyl- oder Arylrest stehen,

n für eine Zahl von 1 bis 4 steht und

A für die Gruppe $-CH=CH-$ oder $-CH_2-CH_2-$ steht,

als solche oder in Form ihrer pharmakologisch unbedenklichen Salze, Verfahren zu ihrer Herstellung sowie ihre Verwendung in Arzneimitteln, insbesondere in antithrombo-embolischen Mitteln.

EP 0 029 139 A1

ACTORUM AG

0029139

BAYER AKTIENGESELLSCHAFT 5090 Leverkusen, Bayerwerk
Zentralbereich
Patente, Marken und Lizenzen KS/kl-c
Ia (Pha)

Imidazolderivate, Verfahren zu ihrer Herstellung sowie ihre Verwendung in Arzneimitteln

Die vorliegende Erfindung betrifft neue Imidazolderivate, Verfahren zu ihrer Herstellung sowie ihre Verwendung in Arzneimitteln, insbesondere in antithromboembolischen Mitteln.

Thrombose und arteriosklerotische Gefäßwandveränderungen werden vor allem durch die Wechselwirkung zweier Metaboliten der Arachidonsäure, nämlich durch das Thromboxan $A_2$ ($TXA_2$) auf der einen und durch das Prostacyclin ($PGI_2$) auf der anderen Seite gesteuert. $TXA_2$ wirkt auf die Blutplättchen aggregierend und $PGI_2$ hat eine antiaggregierende Wirkung. Darüber hinaus wirkt $TXA_2$ vasokonstriktorisch und $PGI_2$ vasodilatatorisch.

Bei einer Reihe von thrombo-embolischen und ischämischen Erkrankungen führt Hyperaggrebilität der Plättchen bzw. ein erhöhter Plättchenverbrauch zu einer gesteigerten Thromboxansynthese, so daß das $TXA_2$- und

$PGI_2$-Gleichgewicht gestört ist. Es ist deshalb zur Therapie und Prophylaxe von thrombo-embolischen und ischämischen Erkrankungen wünschenswert, die Thromboxansynthese zu hemmen und damit die protektive Eigenschaft des $PGI_2$ zu erhöhen.

Man kennt bereits das Imidazol und das N-Methylimidazol, die in gewissem Umfang selektiv die Thromboxansynthese und dadurch die Bildung des $TXA_2$ aus den Prostaglandinendoperoxiden hemmen (Moncada et al., Prostaglandins, Band 13, Seite 611 (1977)).

Weiterhin sind bereits N-Alkylimidazole mit antithromboembolischer Wirkung bekannt geworden (vgl.: Europäische Anmeldung Nr. 951), wobei der Alkylrest auch in cyclischer Form vorliegen kann. Derivate des Imidazols und ihre hemmende Wirkung auf die Thromboxansynthese sind neu.

Die vorliegende Erfindung betrifft Imidazolderivate der allgemeinen Formel (I)

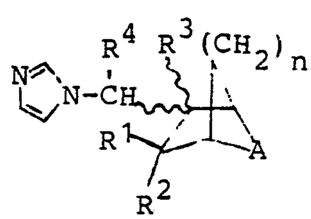

(I)

in welcher

$R^1$, $R^2$, $R^3$ und $R^4$ für Wasserstoff oder einen gegebenenfalls substituierten Alkyl- oder Arylrest stehen,

n für eine Zahl von 1 bis 4 steht und

A für die Gruppe -CH=CH- oder $-CH_2-CH_2-$ steht

als solche oder in Form ihrer pharmakologisch unbedenklichen Salze.

Überraschenderweise besitzen die Imidazolderivate der allgemeinen Formel (I) eine stärkere und spezifischere Hemmwirkung auf die Thromboxansynthese als die aus dem Stand der Technik bekannten Stoffe. Ihre Verwendung in antithromboembolisch wirksamen Arzneimitteln, insbesondere in Arzneimitteln mit thromboxansynthesehemmender Wirkung stellt eine Bereicherung der Pharmazie dar.

Die Herstellung der erfindungsgemäßen Verbindungen erfolgt indem man Imidazole der allgemeinen Formel (II)

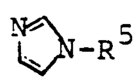

(II)

in welcher

$R^5$ für Wasserstoff oder Alkalimetall steht,

mit Bicyclen der allgemeinen Formel (III)

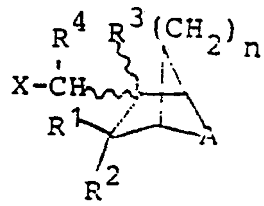

(III)

in welcher

$R^1$, $R^2$, $R^3$, $R^4$, n und A die oben angegebene Bedeutung haben und

X für Halogen oder für einen gegebenenfalls substituierten Alkyl- oder Arylsulfonylsäurerest steht,

in Gegenwart von inerten organischen Lösungsmitteln bei Temperaturen zwischen 0 und 150°C umsetzt.

In der vorliegenden Anmeldung bedeutet die Schreibweise ~~~, daß die jeweiligen Substituenten sowohl exo- als auch endo-ständig angeordnet sein können.

Verwendet man als Ausgangsstoffe Imidazol-natrium und 5-Brommethylbicyclo-[2,2,1]-hept-2-en, so kann der Reaktionsablauf durch folgendes Formelschema wiedergegeben werden:

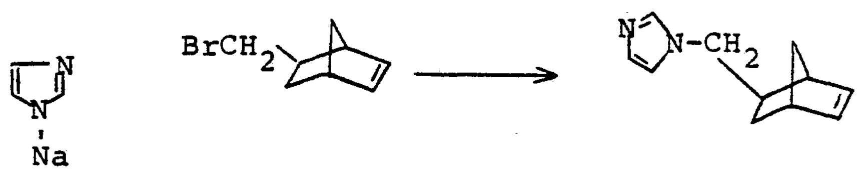

Die als Ausgangsstoffe verwendeten Imidazole der allgemeinen Formel (II) sind bekannt (vgl. Beilstein 23 (2) 35).

In der allgemeinen Formel (II) steht $R^5$ vorzugsweise für Wasserstoff, Lithium, Natrium oder Kalium.

Die ebenfalls als Ausgangsstoffe verwendeten Bicyclen der allgemeinen Formel (III) sind bekannt oder können nach bekannten Methoden hergestellt werden (vgl. Britisches Patent 1 123 878; J. Organic Chemistry, Band 32, Seite 3958 (1967)).

Als Bicyclen der allgemeinen Formel (III) seien beispielhaft genannt:

5-Brommethyl-bicyclo[2,2,1]hept-2-en,
5-Chlormethyl-bicyclo[2,2,1]hept-2-en,
5-Brommethyl-bicyclo[2,2,2]oct-2-en,
6-Methyl-5-brommethyl-bicyclo[2,2,1]hept-2-en,
5-Chlorbenzyl-bicyclo[2,2,1]hept-2-en,
2-Brommethyl-bicyclo[2,2,1]heptan.

In den allgemeinen Formeln (I) und (III) stehen

$R^1$, $R^2$; $R^3$ und $R^4$ vorzugsweise für Wasserstoff oder geradkettiges oder verzweigtes Alkyl mit 1 bis 6 Kohlenstoffatomen, welches gegebenenfalls durch Halogen oder Trifluormethl substituiert ist, oder für Phenyl,

welches gegebenenfalls durch Halogen, Trifluormethyl oder Alkyl mit 1 bis 2 Kohlenstoffatomen substituiert ist,

n vorzugsweise für eine Zahl von 1 bis 3 und

A für die Gruppe CH=CH- oder die Gruppe $-CH_2-CH_2-$.

In der Formel (III) steht X vorzugsweise für Chlor, Brom, Iod oder für einen Alkylsulfonsäurerest mit 1 bis 4 Kohlenstoffatomen, insbesondere für den Methan- oder Ethansulfonsäurerest, oder für einen Phenylsulfonsäurerest der gegebenenfalls durch Alkyl mit 1 bis 2 Kohlenstoffatomen, durch Chlor oder Brom oder durch Nitro substituiert ist, insbesondere für den p-Toluolsulfonsäurerest.

Von besonderem Interesse sind Verbindungen der allgemeinen Formel (I) in welcher

$R^1$, $R^2$, $R^3$ und $R^4$ für Wasserstoff, Methyl, Ethyl oder Phenyl stehen,

n für die Zahl 1 oder 2 steht und

A die oben angegebene Bedeutung hat.

Als Verdünnungsmittel kommen alle inerten organischen Lösungsmittel in Frage. Hierzu gehören vorzugsweise cyclische oder acyclische Ether wie Tetrahydrofuran, Dioxan oder Dimethoxyethan, aliphatische Carbonsäurenitrile wie Acetonitril oder Propionitril, aliphatische N-substituierte Carbonsäureamide wie Dimethylformamid oder Dimethylacetamid, Phosphorsäureamide wie Hexamethylphosphorsäuretriamid und Sulfoxide wie Dimethylsulfoxid.

Es ist nicht notwendig, die Imidazolyl-Metallverbindungen als solche einzusetzen. Es genügt in vielen Fällen, diese im Verdünnungsmittel aus Imidazol und entsprechenden Metallverbindungen herzustellen.

Geeignete Metallverbindungen sind beispielsweise Alkalihydroxide wie Natriumhydroxid und Kaliumhydroxid, Alkalialkoholate, wie Natriummethylat, Kalium-tert.-butylat, Alkalihydride Verbindungen wie Butyllithium, tert.-Butyllithium oder Phenyllithium.

Die Reaktionstemperaturen können in einem größeren Bereich varriert werden. Im allgemeinen arbeitet man zwischen 0 und etwa 150°C, vorzugsweise zwischen 10 und 100°C.

Bei der Durchführung des erfindungsgemäßen Verfahrens setzt man 1 Mol der Verbindung (III) mit 2 bis 6 Mol, vorzugsweise mit 2 bis 4 Mol Imidazol oder bei Verwendung von Imidazoliden mit 0,9 bis 1,1 Mol und vorzugsweise mit 0,95 bis 1,0 Mol Imidazolid um.

Die Reaktionsdauer ist von der Temperatur abhängig und liegt zwischen 1 und 10 Stunden.

Falls erforderlich kann eine Reinigung entweder durch

0029139

Umkristallisation, Destillation oder durch Chromatographie erfolgen.

Als neue Wirkstoffe seien in einzelnen genannt:

N-(Bicyclo[2,2,1]hept-2-en-5-yl-methyl)-imidazol,
N-(Bicyclo[2,2,2]-oct-2-en-5-yl-methyl)-imidazol,
N-(6-Methyl-bicyclo[2,2,1]hept-2-en-5-yl-methyl)-imidazol,
N-(Bicyclo[2,2,1]hept-2-en-5-yl)-benzyl)-imidazol,
N-(Bicyclo[2,2,1]hept-2-yl-methyl)-imidazol.

Die Verbindungen können als exo- oder endo-Isomere oder als Gemische vorliegen.

Als Zubereitungsformen kommen die üblichen galenischen Applikationsformen in Frage, beispielsweise Cremes, Tabletten, Pillen, Kapseln, Suppositorien, Emulsionen, Infusions- und Injektionslösungen. Diese Zubereitungsformen werden nach an sich bekannten Methoden hergestellt unter Verwendung üblicher Hilfs- und Trägerstoffe.

Der Einsatz der so hergestellten Arzneimittel erfolgt je nach Bedarf z.B. durch lokale, parenterale oder orale Verabreichung.

Besonders geeignet sind Formulierungen, die die erfindungsgemäßen Verbindungen von etwa 0,1 bis 10 Gew.-% enthalten. Besonders bevorzugt sind wäßrige Lösungen, die gegebenenfalls auf einen pH-Wert von 6 bis 8 gepuffert sind.

Die Dosierung der erfindungsgemäßen Verbindungen liegt vorzugsweise in einem Bereich von 0,05 bis 100 mg/kg, insbesondere von 0,1 bis 20 mg/kg Körpergewicht.

Beispiel 1

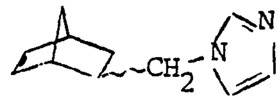

N-(Bicyclo[2,2,1]-hept-2-en-5-yl-methyl)-imidazol

14 g Imidazol löst man in 200 ml absolutem Dimethylformamid und versetzt mit 6 g Natriumhydrid (80 %ig) portionsweise. Man rührt bei 20°C bis zum Ende der Gasentwicklung und läßt dann 37,4 g 5-Brommethyl-bicyclo-[2,2,1]hept-2-en in 100 ml absolutem Dimethylformamid einfließen. Man erwärmt 4 Stunden auf 60°C. Das Dimethylformamid wird im Vakuum abdestilliert, der Rückstand in Essigester aufgenommen. Man trennt den Niederschlag ab, dampft das Lösungsmittel im Wasserstrahlvakuum ab und chromatographiert den Rückstand an Kieselgel mit Methylenchlorid/10 % Methanol. Man erhält 24 g N-(Bicyclo[2,2,1]hept-2-en-5-yl-methyl)-imidazol (Ausbeute: 69 %). $R_f$ = 0,42 (obiges System).

$^1$H-NMR ($CDCl_3$): $\delta$ = 7,45 (s, 1H), 6,95 (d, J = 8 Hz, 2H) und 5,90 - 6,39 ppm (m, 2H).

Beispiel 2

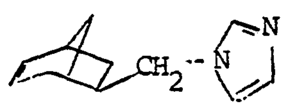

N-(Bicyclo[2,2,2]-oct-2-en-5-yl-methyl)-imidazol

Analog Beispiel 1 erhält man N-(Bicyclo[2,2,2]-oct-2-en-5-yl-methyl)-imidazol in einer Ausbeute von 65 %.
$R_f$ = 0,44 (obiges System).
IR (Film): $\nu$ = 1550 und 1440 $cm^{-1}$.

Beispiel 3

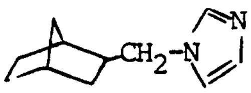

N-(Bicyclo/2,2,1/hept-2-yl-methyl)-imidazol

Analog Beispiel 1 erhält man N-(Bicyclo/2,2,1/hept-2-yl-methyl)-imidazol in einer Ausbeute von 70 %.
$R_f$ = 0,47 (obiges System).
Kp 180-111°C/0,04 mbar.

Patentansprüche

1. Imidazolderivate der allgemeinen Formel (I)

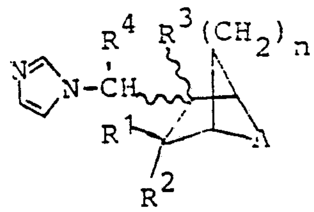

(I)

in welcher

$R^1$, $R^2$, $R^3$ und $R^4$ für Wasserstoff oder einen gegebenefalls substituierten Alkyl- oder Arylrest stehen,

n für eine Zahl von 1 bis 4 steht und

A für die Gruppe -CH=CH- oder $-CH_2-CH_2-$ steht,

als solche oder in Form ihrer pharmakologisch unbedenklichen Salze.

2. Verbindungen der allgemeinen Formel (I) gemäß Anspruch 1, in welcher

$R^1$, $R^2$, $R^3$ und $R^3$ für Wasserstoff, geradkettiges oder verzweigtes Alkyl mit 1 bis 6 Kohlenstffatomen, welches gegebenenfalls durch Halogen oder Trifluormethyl substituiert ist, oder für Phenyl, welches gegebenenfalls durch Halogen,

Trifluormethyl oder Alkyl mit 1 bis 2 Kohlenstoffatomen substituiert ist, stehen

n für eine Zahl von 1 bis 3 steht und

A für die Gruppe -CH=CH- oder für die Gruppe $-CH_2-CH_2-$ steht.

3. Verbindungen der allgemeinen Formel (I) gemäß Anspruch 1, in welcher

$R^1$, $R^2$, $R^3$ und $R^4$ für Wasserstoff, Methyl, Ethyl oder Phenyl stehen,

n für die Zahl 1 oder 2 steht und

A die in Anspruch 1 angegebene Bedeutung hat.

4. Verfahren zur Herstellung von Verbindungen gemäß Anspruch 1, dadurch gekennzeichnet, daß man Imidazole der allgemeinen Formel (II)

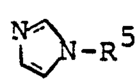

(II)

in welcher

$R^5$ für Wasserstoff oder Alkalimetall steht,

mit Bicyclen der allgemeinen Formel (III)

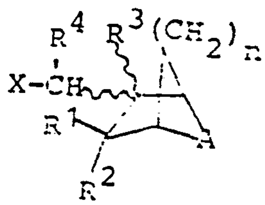

(III)

in welcher

$R^1$, $R^2$, $R^3$, $R^4$, n und A die oben angegebene Bedeutung haben und

X für Halogen oder für einen gegebenenfalls substituierten Alkyl- oder Arylsulfonylsäurerest steht,

gegebenenfalls in Gegenwart von inerten organischen Lösungsmitteln bei Temperaturen zwischen 0 und 150°C umsetzt.

5. Arzneimittel enthaltend mindestens eine Verbindung der allgemeinen Formel (I) gemäß Anspruch 1.

6. Antithromboembolisch wirksames Arzneimittel enthaltend mindestens eine Verbindung der allgemeinen Formel (I) gemäß 1.

7. Verfahren zur Herstellung von Arzneimitteln dadurch gekennzeichnet, daß man Verbindungen der allgemeinen Formel (I) gemäß Anspruch 1 gegebenenfalls unter Verwendung üblicher Hilfs- und Trägerstoffe in eine geeignete Applikationsform überführt.

8. Verwendung von Verbindungen gemäß Anspruch 1 bei der Bekämpfung von thromboembolischen Erkrankungen.

9. Verfahren zur Behandlung von thromboembolischen Erkrankungen, dadurch gekennzeichnet, daß man Verbindungen gemäß Anspruch 1 Menschen oder Tieren im Bedarfsfalle appliziert.

0029139

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 80 10 6576.4

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.3) |
|---|---|---|---|
| P,X | EP - A1 - 0 015 002 (WELLCOME FOUNDATION LTD.)<br>* Ansprüche 1 bis 16 *<br>-- | 1-8 | C 07 D 233/58<br>A 61 K 31/415 |
| P | DE - A1 - 2 855 329 (GRÜNENTHAL GMBH)<br>* Ansprüche 1, 13 *<br>-- | 4,5 | |
| | EP - A1 - 0 000 950 (WELLCOME FOUNDATION LTD.)<br>* Ansprüche 1, 7 *<br>-- | 1,4 | **RECHERCHIERTE SACHGEBIETE (Int. Cl.3)** |
| D | EP - A1 - 0 000 951 (WELLCOME FOUNDATION LTD.)<br>* Ansprüche 1, 11, 12 *<br>---- | 1,5-8 | A 61 K 31/415<br>C 07 D 233/58 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8
Unvollständig recherchierte Patentansprüche: --
Nicht recherchierte Patentansprüche: 9
Grund für die Beschränkung der Recherche:

Verfahren zur therapeutischen Behandlung des menschlichen oder tierischen Körpers

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 16-02-1981 | FROELICH |